# EUROPÄISCHE PATENTSCHRIFT

(11) **EP 3 093 644 B1**
(45) Veröffentlichungstag und Bekanntmachung des Hinweises auf die Patenterteilung: **18.09.2019**
(21) Anmeldenummer: 16161985.3
(22) Anmeldetag: 23.03.2016
(51) Int. Cl.: G01N 1/04, G01N 30/00, G01N 27/62

(54) **OBERFLÄCHENUNTERSUCHUNG VON BAUTEILEN**
INVESTIGATION OF THE SURFACES OF COMPONENTS
INSPECTION DE SURFACE DE COMPOSANTS

(30) Priorität: 11.05.2015 DE 102015107342
(43) Veröffentlichungstag der Anmeldung: 16.11.2016
(73) Patentinhaber: Airbus Defence and Space GmbH, 85521 Ottobrunn (DE)
(72) Erfinder: Wachinger, Georg, 85521 Ottobrunn (DE); Helwig, Dr. Andreas, 85521 Ottobrunn (DE); Meer, Thomas, 85521 Ottobrunn (DE); Geistbeck, Dr. Matthias, 85521 Ottobrunn (DE); Friedberger, Dr. Alois, 85521 Ottobrunn (DE); Heckner, Sebastian, 85521 Ottobrunn (DE); Göbel, Johann, 85521 Ottobrunn (DE)
(74) Vertreter: Isarpatent

(56) Entgegenhaltungen:
- EP-A1- 0 897 108
- WO-A1-2008/115855
- DE-A1-102011 102 055
- F Li ET AL: "Ion mobility spectrometer for online monitoring of trace compounds", Spectrochimica Acta Part B: Atomic Spectroscopy, 1. Januar 2002 (2002-01-01), Seiten 1563-1574, XP055299684, DOI: 10.1016/S0584-8547(02)00110-6 Gefunden im Internet: URL:http://ac.els-cdn.com/S058485470200110 6/1-s2.0-S0584854702001106-main.pdf?_tid=2 72c5598-711f-11e6-955b-00000aab0f26&acdnat =1472829109_31d600f3918a44bc5fae0fa05a5843 26 [gefunden am 2016-09-02]

## Beschreibung

Die vorliegende Erfindung betrifft eine Vorrichtung und ein Verfahren zur Oberflächenuntersuchung von Bauteilen.

Während der Herstellung von Bauteilen, insbesondere von Elementen aus Faserverbundkunststoffen wie beispielsweise kohlenstofffaserverstärktem Kunststoff, werden häufig Fertigungshilfsmittel verwendet, die nach der Fertigung auf der Oberfläche des Bauteils zurückbleiben. Dies betrifft beispielsweise Trennmittel, die nach dem Entformen des jeweiligen Bauteils als Rückstand auf der Oberfläche haften bleiben können.

Für die weitere Verarbeitung kann das Vorhandensein derartiger Rückstände nachteilig sein. Beispielsweise wird beim strukturellen Kleben die Festigkeit einer Klebung negativ von oberflächlichen Kontaminationen, insbesondere Trennmittelrückständen beeinflusst. Als Konsequenz kann das verarbeitete Produkt Qualitätsmängel aufweisen.

Um feststellen zu können, ob ein Bauteil sich für eine bestimmte Verarbeitung (wie das strukturelle Kleben) eignet und um ggf. Gegenmaßnahmen treffen zu können, wird daher ein Prüfverfahren benötigt, mit dem erkannt werden kann, ob, womit und in welchem Ausmaß eine Bauteiloberfläche gegebenenfalls verunreinigt ist.

Direkte Messungen von Kontaminationen in flüssiger oder fester Form an der Oberfläche sind dabei aufgrund der zumeist sehr geringen Schichtdicken der Verunreinigungen problematisch.
Wesentlich sensitiver sind Messtechniken, die in der Gasphase messen.

In der DE 10 2011 102 055 A1 ist insbesondere eine Vorrichtung zur Prüfung eines Faserverbundbauteils auf Kontaminationen offenbart. Diese Apparatur umfasst insbesondere eine Oberflächenerwärmungsvorrichtung und ein Sensorarray zur Erfassung von Kontaminationsstoffen, die von der erwärmten Oberfläche des Faserverbundteils desorbiert wurden.

Weiterhin ist beispielsweise die laserinduzierte Plasmaspektroskopie (kurz: "LIBS") bekannt. Mit dieser Technologie kann das chemische Element Silikon in Siloxanbasierten Kontaminationen detektiert werden. Ein Nachteil dieser Technik ist jedoch, dass es mit ihr nicht möglich ist, zwischen Silikonen und Silikaten zu unterscheiden. Für die Beurteilung der Oberfläche in Bezug auf nachfolgende Klebevorgänge ist diese Unterscheidung jedoch bedeutsam.

Eine Analyse mit Hilfe eines Fourier-Transformations-Infrarotspektrometers (FTIR) ist hingegen zur Erfassung von Trennmittelresten nicht geeignet, weil die auftretenden Konzentrationen zu gering sind, um auf diese Weise gemessen werden zu können.

Die WO 2008/115855 A1 beschreibt eine Vorrichtung zum Erwärmen einer Oberfläche, um mindestens einen Analyten zum Nachweis derselben freizusetzen mit einer Energiequelle zum Bestrahlen der Oberfläche und einem Kollektor zum Sammeln mindestens eines Gases von der Oberfläche, wobei das mindestens eine Gas in der Lage ist, den mindestens einen freigesetzten Analyten mitzuführen. Die Vorrichtung beinhaltet ferner einen Detektor, der mit dem Kollektor verbunden ist, um das Vorhandensein des mindestens einen freigesetzten Analyten zu erfassen, wobei der Nachweis verwendet wird, um die Leistung der Energiequelle durch Verwendung von Rückkopplungen in Bezug auf mindestens einen Zustand der Oberfläche zu steuern.

Die vorliegende Erfindung hat daher die Aufgabe, eine Technik bereitzustellen, die es insbesondere ermöglicht, Trennmittelreste auf Faserverbundkunststoffen zu detektieren, und mit der die genannten Nachteile behoben werden.

Die Aufgabe wird gelöst durch eine Vorrichtung gemäß Anspruch 1 und ein Verfahren gemäß Anspruch 6. Bevorzugte Ausführungsformen sind in den Unteransprüchen offenbart.

Eine erfindungsgemäße Vorrichtung dient einer Oberflächenuntersuchung von Faserverbundkunststoff-Bauteilen. Sie umfasst eine Strahlungsquelle, die dazu eingerichtet ist, durch Bestrahlen einer Probenfläche eines Faserverbundkunststoff-Bauteils mindestens einen schwerflüchtigen Stoff zumindest teilweise in Gas umzuwandeln, eine Detektoreinheit, die dazu eingerichtet ist, den mindestens einen in Gas umgewandelten Stoff qualitativ und/oder quantitativ zu erfassen. Die Vorrichtung umfasst weiterhin eine Messglocke, die dazu eingerichtet ist, um das Faserverbundkunststoff-Bauteil herum oder an dem Faserverbundkunststoff-Bauteil anliegend ein Messvolumen auszubilden, das an die Probenfläche angrenzt. Die Messglocke umfasst eine Abdichtung aus einem flexiblen Material, die das Messvolumen an einem Übergang zwischen der Messglocke un der Probenfläche abdichtet. Die Strahlungsquelle ist dazu eingerichtet, die Probenfläche durch ein energiedurchlässiges Einkoppelfenster in der Messglocke hindurch zu bestrahlen, wobei die Messglocke ein solches Einkoppelfenster umfasst. Die Vorrichtung umfasst eine Prozessüberwachungseinheit, die dazu eingerichtet ist, eine an der Probenfläche herrschende Temperatur zu erfassen. Die Strahlungsquelle umfasst einen Regler, der dazu eingerichtet ist, die erfasste Temperatur mit einem vorgegebenen Schwellwert zu vergleichen, welcher eine Gefahr einer thermischen Schädigung der Probefläche impliziert und das Bestrahlen bei Überschreiten der Temperatur über den Schwellwert zu beenden oder in Intensität zu reduzieren. Die Strahlungsquelle umfasst mindestens einen Laser und/oder mindestens einen Plasmastrahler. Die Strahlungsquelle ist dazu eingerichtet, einen Abtrag von Material des Faserverbundkunststoff-Bauteils aus der Probenfläche herauszulösen.

Ein erfindungsgemäßes Verfahren dient einer Oberflächenuntersuchung von Faserverbundkunststoff-Bauteilen. Es umfasst ein Ausbilden eines Messvolumens, das an eine Probenfläche eines Faserverbundkunststoff-Bauteils angrenzt, durch eine Messglocke, die dazu eingerichtet ist, um das Faserverbundkunststoff-Bauteil herum oder an dem Faserverbundkunststoff-Bauteil anliegend das Messvolumen auszubilden, da an die Probenfläche angrenzt. Die Messglocke umfasst eine Abdichtung, die das Messvolumen an einem Übergang zwischen der Messglocke un der Probenfläche abdichtet. Das Verfahren umfasst weiterhin ein Bestrahlen der Probenfläche des Faserverbundkunststoff-Bauteils mit einer Strahlungsquelle, die dazu eingerichtet ist, mindestens einen schwerflüchtigen Stoff zumindest teilweise in Gas umzuwandeln. Das Verfahren umfasst weiterhin ein qualitatives und/oder quantitatives Erfassen des mindestens einen in Gas umgewandelten Stoffs mittels einer Detektoreinheit, wobei durch die Strahlungsquelle die Probenfläche durch ein energiedurchlässiges Einkoppelfenster in der Messglocke hindurch bestrahlt wird, wobei die Messglocke ein solches Einkoppelfenster umfasst. Die Vorrichtung umfasst eine Prozessüberwachungseinheit, durch die eine an der Probenfläche herrschende Temperatur erfasst wird. Die Strahlungsquelle umfasst einen Regler, durch den die erfasste Temperatur mit einem vorgegebenen Schwellwert verglichen wird, welcher eine Gefahr einer thermischen Schädigung der Probefläche impliziert und durch den das Bestrahlen bei Überschreiten der Temperatur über den Schwellwert beendet oder in Intensität reduziert wird. Die Strahlungsquelle umfasst mindestens einen Laser und/oder mindestens einen Plasmastrahler. Die Strahlungsquelle löst einen Abtrag von Material des Faserverbundkunststoff-Bauteils aus der Probenfläche heraus.

Als "schwerflüchtig" wird in dieser Schrift ein Stoff bezeichnet, dessen Verdunstungszahl gemäß DIN 53170 mindestens 35 ist.

Als "Bauteil" ist in dieser Schrift ein Element zu verstehen, das sich für eine weitere Verarbeitung in einem Herstellungsprozess eignet oder das bereits ein fertiges Produkt darstellt. Insbesondere umfasst der Begriff vorgeformte Elemente, die für die Herstellung eines Produktes aneinandergefügt und miteinander verbunden werden können, beispielsweise im Fahrzeugbau, insbesondere im Luftfahrzeugbau. Auch eine Baustoffeinheit wie beispielsweise eine Platte eines Faserverbundkunststofflaminats wird in dieser Schrift von dem Begriff "Bauteil" eingeschlossen. Besonders bevorzugt ist eine Ausführungsform, bei der das Bauteil ein Verbundbauteil eines Luft- oder Raumfahrzeugs ist.

Die Probenfläche kann jeweils eine Gesamtoberfläche des Bauteils oder ein lokaler Oberflächenabschnitt des Bauteils sein; an ihr erfolgt eine Untersuchung des Bauteils.

Eine erfindungsgemäße Vorrichtung und ein erfindungsgemäßes Verfahren ermöglichen es, auf materialschonende Weise eine Vielzahl von schwerflüchtigen Kontaminationen (insbesondere hochmolekulare Verarbeitungsrückstände) zu messen, die mit herkömmlichen Methoden nicht oder nur unter Inkaufnahme einer thermischen Schädigung der Bauteiloberfläche erfasst werden können. Insbesondere erlaubt die vorliegende Erfindung eine Erfassung von schwerflüchtigen Stoffen mit niedrigen Dampfdrücken auch in geringen Konzentrationen oder Mengen.

Die Strahlungsquelle kann beispielsweise mindestens eine LED-Einheit, mindestens einen Infrarotstrahler und/oder mindestens eine Terahertz-Strahlungsquelle umfassen.

Erfindungsgemäß umfasst die Strahlungsquelle mindestens einen Laserstrahler (der insbesondere im Dauerstrichbetrieb und/oder gepulst eingesetzt werden kann) und/oder mindestens einen Plasmastrahler; beide Varianten sind insbesondere auch zur Durchführung einer abtragenden Vorbehandlung wie weiter unten erwähnt besonders geeignet.

Gemäß einer bevorzugten Ausführungsform der vorliegenden Erfindung erfolgt das Bestrahlen während eines Zeitraums von höchstens 180 Sekunden, bevorzugter höchstens 60 Sekunden, noch bevorzugter unter 1 Sekunde. Vorzugsweise wird dabei durch das Bestrahlen eine Temperatur von mindestens 200°C, bevorzugter mindestens 400°C, noch bevorzugter mindestens 500°C an der Probenfläche erzeugt. Eine derartige kurzzeitige Bestrahlung mit hohem Energieeintrag schont das Material und ist dabei gleichwohl effektiv im Umwandeln schwerflüchtiger Stoffe in Gas. Das Gas kann den reinen schwerflüchtigen Stoff in seiner Gasphase umfassen. Alternativ oder zusätzlich kann das Gas ein Abbauprodukt wie beispielsweise ein flüchtiges Verbrennungsprodukt (z.B. Abgas) umfassen, die sich infolge der Bestrahlung entwickelt haben.

Dem entsprechend kann das Erfassen des mindestens einen in Gas umgewandelten Stoffes ein Erfassen mindestens eines bei der Umwandlung entstandenen Abbauprodukts, insbesondere eines flüchtigen Verbrennungsprodukts (z.B. Abgases) umfassen. Insbesondere kann die Bestrahlung für eine Vorbehandlung der Probenfläche eingesetzt werden, beispielsweise in Form einer Materialabtragung zur Reinigung und/oder Aktivierung der Probenfläche. Über das erfindungsgemäße Erfassen von dabei entstandenen flüchtigen Abbauprodukten kann die Vorbehandlung überwacht werden. Zudem können insbesondere die herkömmlich in zwei separaten Verfahren vorgenommenen Prozesse von Trennmittelabtrag und Oberflächenaktivierung kombiniert werden, was eine verfahrenstechnische Vereinfachung und eine Zeitersparnis bedeutet.

Vorzugsweise ist die Strahlungsquelle dazu eingerichtet, außer dem mindestens einen schwerflüchtigen auch mindestens einen weiteren Stoff, insbesondere z.B. mindestens einen leichtflüchtigen Stoff (Verdunstungszahl gemäß DIN 53170 kleiner als 10) in Gas umzuwandeln. Insbesondere kann das Umwandeln des mindestens einen schwerflüchtigen Stoffs mit einem Umwandeln mindestens eines weiteren (z.B. leicht- oder mittelflüchtigen) Stoffs einhergehen.

Besonders bevorzugt ist eine Ausführungsform, bei der die Detektoreinheit mindestens ein Gaschromatographie-Ionenmobilitätsspektrometer (kurz "GC-IMS") umfasst. Eine derartige Detektoreinheit ist insbesondere zur Erfassung sehr kleiner Stoffmengen sowie zum Nachweis von Stoffgemischen geeignet.

Erfindungsgemäß umfasst eine erfindungsgemäße Vorrichtung eine Messglocke, die dazu eingerichtet ist, um das Bauteil herum oder an dem Bauteil anliegend ein Messvolumen auszubilden, das an die Probenfläche angrenzt.

Gemäß einem erfindungsgemäßen Verfahren wird analog die Probenfläche unter einer derartigen Messglocke bestrahlt.

Die Probenfläche bildet zusammen mit der Messglocke und ggf. mit einer weiteren Fläche (beispielsweise einer Unterlage) eine Begrenzung des (um- oder anliegenden) Messvolumens. Beispielsweise kann die Messglocke in einer beliebigen Richtung auf die Probenfläche des Bauteils oder eine Unterlage gestülpt sein, oder sie kann das Bauteil ganz umschließen. Insbesondere ist der präpositionale Ausdruck "unter einer Messglocke" nicht als auf eine vertikale Ausrichtung hinweisend zu interpretieren.

Das Messvolumen kann einen beliebig geformten geometrischen Raum ausbilden (wie beispielsweise eine Kugel, eine Halbkugel, einen Zylinder, einen Kegelstumpf oder einen Quader, um nur einige zu nennen). Insbesondere ist der Begriff der das Messvolumen definierenden "Messglocke" nicht als Beschränkung der Geometrie des Messvolumens zu verstehen.

Bei derartigen, eine Messglocke umfassenden Ausführungsformen kann die Genauigkeit verbessert werden, mit der der mindestens eine in Gas umgewandelte Stoff erfasst wird, weil dieser Stoff mindestens zu einem Teil im Messvolumen aufgefangen wird und sich somit höchstens begrenzt in der Umgebung verteilt.

Darüber hinaus kann eine derartige Messglocke ein Austreten von gesundheitsschädlichem und/oder schlecht riechendem Gas verhindern, das sich durch Bestrahlung entwickelt haben kann.

Die Messglocke umfasst erfindungsgemäß eine Abdichtung aus einem flexiblen Material, die das Messvolumen an einem Übergang zwischen der Messglocke und der Probenfläche abdichtet. Besonders bei Verwendung zur Untersuchung eines Bauteils mit gekrümmter Oberfläche verbessert eine derartige Abdichtung eine Anpassung an eine jeweilige Unterlage, so dass ein unkontrollierter Austausch von Gas mit der Umgebung verhindert wird.

Gemäß einer bevorzugten Ausführungsform ist die Detektoreinheit außerhalb des Messvolumens angeordnet. Vorzugsweise wird der mindestens eine in Gas umgewandelte Stoff durch einen in der Messglocke befindlichen Gasauslass aus dem Messvolumen zur Detektoreinheit geleitet oder gepumpt und dort erfasst. Diese Ausführungsform hat insbesondere den Vorteil, dass die Messglocke entsprechend klein und flexibel einsetzbar ausgebildet sein kann, und dass als Detektoreinheit gleichwohl ein spezifisches Analysegerät (insbesondere z.B. ein Gaschromatographie-Ionenmobilitätsspektrometer) verwendet werden kann. Zudem kann der in Gas umgewandelte Stoff in einer derart separierten Detektoreinheit analysiert werden, ohne dass der durch das Bestrahlen der Probenfläche hervorgerufene Umwandlungsprozess oder auch die Probenfläche selbst beeinträchtigt würde.

Gemäß einer bevorzugten Ausführungsform der vorliegenden Erfindung ist die Messglocke mindestens ein Teil eines tragbaren Handgeräts. Sie hat vorzugsweise einen Durchmesser von maximal 50 cm, bevorzugter maximal 3 cm, noch bevorzugter maximal 1 cm, und/oder eine Masse von höchstens 5 kg, bevorzugter von höchstens 1 kg, noch bevorzugter von höchstens 100 g.

Besonders bevorzugt ist eine Ausführungsform einer erfindungsgemäßen Vorrichtung, die eine regelbare Gaszuleitung umfasst, die dazu eingerichtet ist, das Messvolumen mit einem Versuchsgas zu spülen; dem entsprechend umfasst ein erfindungsgemäßes Verfahren analog ein Spülen des Messvolumens mit Versuchsgas. Auf diese Weise kann die Messgenauigkeit erhöht und/oder der Messprozess gesteuert werden. Als derartiges Versuchsgas eignen sich insbesondere Umgebungsluft, synthetische Luft oder auch ein Inertgas wie z.B. Stickstoff oder Argon.

Zudem kann eine gezielte Regelung einer Gaszufuhrmenge und einer Gasabzugsmenge eine Falschluftzufuhr verhindern. Damit kann insbesondere die Messgenauigkeit erhöht werden, auch wenn das Messvolumen nicht vollständig nach außen hin abgedichtet ist.

Erfindungsgemäß ist die Strahlungsquelle dazu eingerichtet, die Probenfläche durch ein energiedurchlässiges Einkoppelfenster in der Messglocke hindurch zu bestrahlen; das energiedurchlässige Einkoppelfenster schließt somit das Messvolumen gegenüber einer Umgebung der Messglocke ab. Analog erfolgt das Bestrahlen gemäß eines erfindungsgemäßen Verfahrens durch ein derartiges Einkoppelfenster hindurch.

Das energiedurchlässige Einkoppelfenster ermöglicht einen Eintritt von seitens der Strahlungsquelle abgegebener Strahlungsenergie in das Messvolumen. Insbesondere ist es vorzugsweise dazu eingerichtet, die Art und Intensität der eingesetzten Strahlung durchzulassen, die für das mindestens teilweise Umwandeln des mindestens einen Stoffs in Gas erforderlich ist. Dies kann beispielsweise durch ein Material und/oder eine Dicke realisiert sein, das bzw. die auf die Strahlungsquelle und den zu erfassenden (mindestens einen) Stoff abgestimmt ist/ sind.

Die Strahlungsquelle kann somit außerhalb der Messglocke positioniert werden. Durch eine geeignete Entfernung der Strahlungsquelle von der Probenfläche kann so eine breite Streuung der Strahlung erreicht werden, ohne dass das Messvolumen entsprechend groß gewählt werden muss, was eine Genauigkeit der Erfassung von in Gas umgewandeltem Stoff im Messvolumen mindern würde.

Erfindungsgemäß umfasst eine erfindungsgemäßen Vorrichtung eine Prozessüberwachungseinheit, die dazu eingerichtet ist, eine an (insbesondere auf) der Probenfläche herrschende Temperatur zu erfassen; analog umfasst ein erfindungsgemäßes Verfahren ein derartiges Erfassen einer an (bzw. auf) der Probenfläche herrschenden Temperatur. Die Temperatur kann dabei z.B. auf Grundlage einer Temperaturmessung in einem Abstand von der Probenfläche bestimmt werden, beispielsweise in einem Abstand von höchstens 10 cm, bevorzugter von höchstens 2 cm, noch bevorzugter höchstens 0,5 cm von der Probenfläche.

Basierend auf den von der Prozessüberwachungseinheit gemessenen Werten können die Strahlungsquelle und/oder die Detektoreinheit gesteuert werden. Insbesondere kann eine Strahlungsintensität in Abhängigkeit von einer durch die Prozessüberwachungseinheit erfassten Temperatur eingestellt und/oder variiert werden. Dadurch kann ggf. einerseits die Umwandlung des Stoffes in Gas optimiert und andererseits eine thermische Schädigung der Probenfläche vermieden werden.

Eine erfindungsgemäße Vorrichtung ist vorzugsweise mindestens teilweise als tragbares Handgerät ausgebildet oder als Teil eines tragbaren Handgeräts. Insbesondere für eine stichprobenartige Untersuchung des Bauteils an mehreren Stellen kann eine derartige Vorrichtung leicht an dem Bauteil umpositioniert werden, wohingegen das Bauteil selbst an seiner Position belassen werden kann. Dies ist insbesondere bei einem großen Bauteil vorteilhaft, das schwierig zu bewegen ist.

Gemäß einer vorteilhaften Ausführungsvariante eines erfindungsgemäßen Verfahrens ist die Probenfläche eine erste Probenfläche, und umfasst das Verfahren weiterhin ein Bestrahlen einer zweiten Probenfläche des Bauteils mit der Strahlungsquelle zum zumindest teilweisen Umwandeln des mindestens einen schwerflüchtigen Stoffes in ein Gas sowie ein qualitatives und/oder quantitatives Erfassen des umgewandelten Stoffs mit der Detektoreinheit.

Vorzugsweise umfasst das Verfahren weiterhin eine Interpolation von so erfassten Werten zur Bestimmung einer Verteilung des schwerflüchtigen Stoffes auf einer Oberfläche des Bauteils.

Insbesondere kann somit stichprobenartig eine Verunreinigung gemessen werden, und es können Rückschlüsse hinsichtlich einer Kontamination einer über die untersuchten Probenflächen hinausgehenden Bauteiloberfläche gezogen werden. Auf diese Weise kann beispielsweise bestimmt werden, ob das Bauteil sich für eine strukturelle Klebung eignet, z.B. weil die Verunreinigung unterhalb eines vorbestimmten Schwellwertes liegt.

Im Folgenden werden bevorzugte Ausführungsbeispiele der Erfindung anhand von Zeichnungen näher erläutert. Es versteht sich, dass einzelne Elemente und Komponenten auch anders kombiniert werden können als dargestellt.

Es zeigen schematisch:
- Figur 1: eine Vorrichtung gemäß einer beispielhaften Ausführungsform der vorliegenden Erfindung während einer ersten Untersuchung; und
- Figur 2: die Vorrichtung gemäß Figur 1 während einer zweiten Untersuchung.

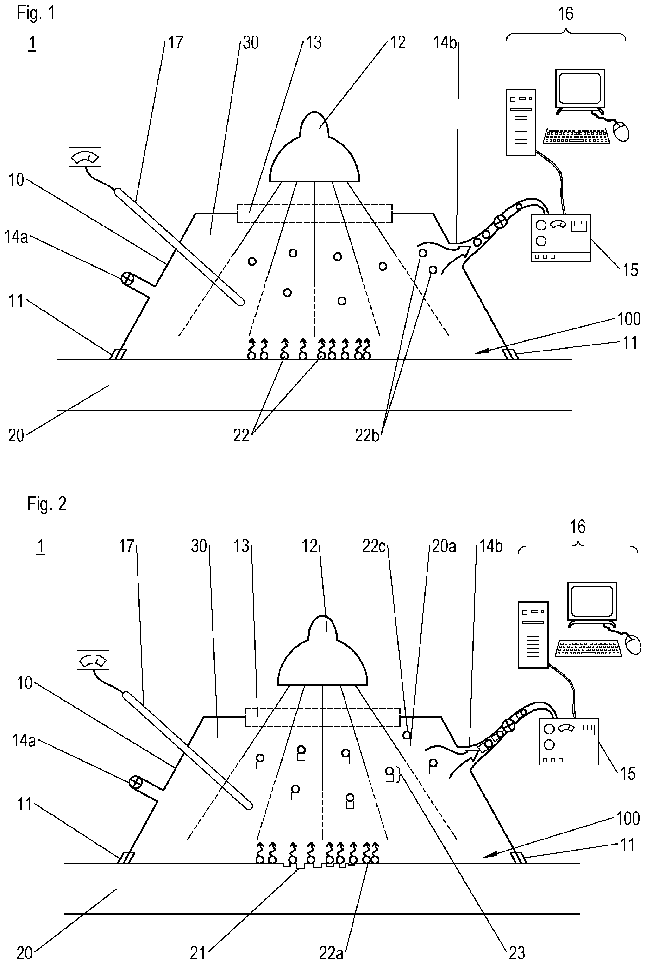

In Figur 1 ist (nicht maßstabsgetreu) eine Anordnung schematisch dargestellt, die eine Ausführungsform einer erfindungsgemäßen Vorrichtung 1 umfasst.

Die Vorrichtung umfasst eine Messglocke 10, die an einem Bauteil 20 anliegend ein Messvolumen 30 ausbildet. Insbesondere wird das Messvolumen 30 von der Messglocke und einer Probenfläche 100, die Teil einer Oberfläche des Bauteils 20 ist, eingeschlossen.

Die Messglocke 10 weist an ihrem Rand eine Abdichtung 11 auf, die das Messvolumen an einem Übergang zwischen der Messglocke 10 und der Probenfläche 100 abdichtet.

Auf dem Bauteil 20, das beispielsweise Faser-Matrix-Verbundmaterial umfassen kann, ist im dargestellten Beispiel ein schwerflüchtiger Stoff 22a abgelagert, der eine Kontamination in Form einer festen, zähflüssigen oder flüssigen Schicht darstellt (die beispielsweise hochmolekulare Trennmittelrückstände umfassen kann).

Die Vorrichtung 1 umfasst weiterhin eine Strahlungsquelle 12, die dazu eingerichtet ist, die Probenfläche 100 zu bestrahlen und dadurch den schwerflüchtigen Stoff 22a zumindest teilweise in Gas 22b umzuwandeln. Im dargestellten Ausführungsbeispiel ist die Strahlungsquelle außerhalb der Messglocke 10 angeordnet, und sie bestrahlt die Probenfläche durch ein in der Messglocke 10 befindliches energiedurchlässiges Fenster 13.

Eine Gaszufuhrregelung umfasst eine Gaszuleitung 14a und ist dazu eingerichtet, ein vorbestimmtes Versuchsgas (beispielsweise Umgebungsluft oder ein Inertgas) durch das Messvolumen 30 einzuleiten und so den Messprozess zu steuern.

Durch einen Gasauslass 14b in der Messglocke hindurch wird (insbesondere) der mindestens eine in Gas umgewandelte Stoff im dargestellten Ausführungsbeispiel zu einer Detektoreinheit 15 (mit einem oder mehreren Sensoren) geleitet oder gepumpt, die dazu eingerichtet ist, den mindestens einen in Gas umgewandelten Stoff 22b qualitativ und/oder quantitativ zu erfassen; bevorzugt ist eine Ausführungsform, bei der die Detektoreinheit mindestens ein Gaschromatographielonenmobilitätsspektrometer umfasst.

Gaszuleitung 14a und Gasauslass 14b können zudem (z.B. während einer Unterbrechung der Bestrahlung und/oder Erfassung) zu einem Spülen des Messvolumens 30 und der Detektoreinheit 15 mit Versuchsgas und/oder Umgebungsluft und oder synthetischer Luft verwendet werden. So kann eine Genauigkeit einer nachfolgenden Messung (mit Bestrahlung und Erfassung wie beschrieben) erhöht werden.

Die Detektoreinheit 15 ist im gezeigten Ausführungsbeispiel an eine Rechnereinheit 16 angeschlossen, die dazu eingerichtet ist, die von der Detektoreinheit 15 gemessenen Werte auszuwerten. Insbesondere kann die Rechnereinheit dazu eingerichtet sein, an verschiedenen Probenflächen erfasste Werte zu interpolieren und eine Verteilung des mindestens einen schwerflüchtigen Stoffes auf einer die Probenflächen und mindestens eine weitere Fläche umfassenden Oberfläche (insbesondere beispielsweise auf der gesamten Oberfläche des Bauteils 20) zu bestimmen.

Eine Prozessüberwachungseinheit 17 ist dazu eingerichtet, eine an der Probenfläche herrschende Temperatur zu erfassen. Gemäß einer bevorzugten Ausführungsform ist die Prozessüberwachungseinheit mit einem Regler der Strahlungsquelle 12 und/oder mit der Gaszuleitung 14a und/oder mit der Detektoreinheit 15 und/oder der Rechnereinheit 16 verbunden und werden die von der Prozessüberwachungseinheit gemessenen Temperaturen zur Steuerung der Strahlungsquelle 12 bzw. zur Steuerung der Gaszuleitung bzw. zur Auswertung der von der Detektoreinheit erfassten Werte herangezogen. Insbesondere kann die erfasste Temperatur mit einem vorgegebenen Schwellwert verglichen werden, der eine Gefahr einer thermischen Schädigung der Probenfläche 100 impliziert. So kann die Bestrahlung beendet oder in der Intensität der Strahlung reduziert werden, wenn die von der Prozessüberwachungseinheit 17 erfasste Temperatur den Schwellwert überschreitet. Alternativ oder zusätzlich kann eine Intensität der Strahlung im Sinne einer Optimierung der Umwandlung des schwerflüchtigen Stoffs in Gas gesteuert werden.

In Verwendung der Vorrichtung wird nun die Probenfläche 100 mit Hilfe der Strahlungsquelle 12 bestrahlt. Dadurch wird der schwerflüchtige Stoff 22a mindestens teilweise in ein Gas 22b umgewandelt, wie durch die Pfeile dargestellt. Die Prozessüberwachungseinheit 17 erfasst dabei vorzugsweise die an der Probenfläche herrschende Temperatur; abhängig vom erfassten Wert kann die Strahlungsquelle entsprechend gesteuert werden wie oben beschrieben.

Die Detektoreinheit 15 erfasst den in Gas umgewandelten Stoff, vorzugsweise mehrere Male, und leitet die jeweils erfassten Werte an die Recheneinheit 16 weiter, die diese Werte für eine Bestimmung einer Kontamination der Probenfläche mit dem schwerflüchtigen Stoff 22a auswertet, beispielsweise durch Vergleiche mit simulierten und/oder gespeicherten Daten.

In Figur 2 ist eine analoge Vorrichtung 1 in einer alternativen Verwendung gezeigt; gleiche Bestandteile sind dabei mit denselben Bezugszeichen versehen wie in Figur 1, auf deren obige Beschreibung verwiesen wird.

In der in Figur 2 gezeigten Verwendung bewirkt die Bestrahlung einen oberflächigen Abtrag des Bauteilmaterials, der schematisch durch Vertiefungen 21 in der Probenfläche dargestellt ist. Das entstandene Abgas 23 umfasst neben dem in ein Gas umgewandelten schwerflüchtigen Stoff 22c daher Abbauprodukte 20a des Bauteilmaterials. Dieses Abgas 23 wird von der Detektoreinheit 15 quantitativ und/oder qualitativ erfasst.

Sowohl der in Figur 1 dargestellte Prozess als auch die in Figur 2 dargestellte Verwendung kann jeweils über einen vorgegebenen Zeitraum (beispielsweise von etwa 1 min oder etwa 10 min oder etwa 30 min) fortgesetzt werden. Alternativ kann eine Dauer des Verfahrens in Abhängigkeit von Werten gesteuert sein, die von der Detektoreinheit erfasst wurden.

Beispielsweise kann die Bestrahlung als eine Gegenmaßnahme für die Kontamination (und damit als Vorbehandlung für einen weiteren Verarbeitungsschritt wie beispielsweise ein strukturelles Kleben des Bauteils) eingesetzt werden, bei der die Bestrahlung einem Abtragen des mindestens einen schwerflüchtigen Stoffs dient. Dabei kann die Bestrahlung beispielsweise fortgesetzt oder iteriert werden, bis der von der Detektoreinheit erfasste Wert für den in Gas umgewandelten Stoff einen Schwellwert unterschreitet; danach wird der Prozess vorzugsweise beendet. Der Schwellwert kann dabei beispielsweise so gewählt sein, dass er eine Toleranzgrenze angibt, unterhalb der eine Kontamination eine weitere Verarbeitung nicht oder nur unwesentlich beeinträchtigt.

### Bezugszeichen

- 1: Vorrichtung
- 10: Messglocke
- 11: Abdichtung
- 12: Strahlungsquelle
- 13: Fenster
- 14a: Gaszuleitung
- 14b: Gasauslass
- 15: Detektoreinheit
- 16: Rechnereinheit
- 20: Bauteil
- 20a: Abbauprodukt
- 21: Vertiefung
- 22a: schwerflüchtiger Stoff
- 22b, 22c: in Gas umgewandelter Stoff
- 23: Abgas
- 30: Messvolumen
- 100: Probenfläche

## Patentansprüche

1. Vorrichtung (1) zur Oberflächenuntersuchung von Faserverbundkunststoff-Bauteilen, die umfasst:
eine Strahlungsquelle (12), die dazu eingerichtet ist, durch Bestrahlen einer Probenfläche (100) eines Faserverbundkunststoff-Bauteils (20) mindestens einen schwerflüchtigen Stoff (22a) zumindest teilweise in Gas umzuwandeln;
eine Detektoreinheit (15), die dazu eingerichtet ist, den mindestens einen in Gas umgewandelten Stoff (22b, 22c, 23) qualitativ und/oder quantitativ zu erfassen, wobei die Vorrichtung zudem eine Messglocke (10) umfasst, die dazu eingerichtet ist, um das Faserverbundkunststoff-Bauteil herum oder an dem Faserverbundkunststoff-Bauteil anliegend ein Messvolumen (30) auszubilden, das an die Probenfläche (100) angrenzt, wobei die Messglocke eine Abdichtung (11) aus einem flexiblen Material umfasst, die das Messvolumen an einem Übergang zwischen der Messglocke (10) und der Probenfläche (100) abdichtet,
wobei die Strahlungsquelle (12) dazu eingerichtet ist, die Probenfläche (100) durch ein energiedurchlässiges Einkoppelfenster (13) in der Messglocke (10) hindurch zu bestrahlen, wobei die Messglocke (10) ein solches Einkoppelfenster (13) umfasst,
die Vorrichtung (1) eine Prozessüberwachungseinheit (17) umfasst, die dazu eingerichtet ist, eine an der Probenfläche (100) herrschende Temperatur zu erfassen und wobei die Strahlungsquelle (12) einen Regler umfasst, der dazu eingerichtet ist, die erfasste Temperatur mit einem vorgegebenen Schwellwert zu vergleichen, welcher eine Gefahr einer thermischen Schädigung der Probefläche (100) impliziert und das Bestrahlen bei Überschreiten der Temperatur über den Schwellwert zu beenden oder in Intensität zu reduzieren, umfasst, und wobei
die Strahlungsquelle (12) mindestens einen Laser und/oder mindestens einen Plasmastrahler umfasst,
**dadurch gekennzeichnet, dass**
die Strahlungsquelle (12) dazu eingerichtet ist, einen Abtrag (20a) von Material des Faserverbundkunststoff-Bauteils aus der Probenfläche (100) herauszulösen.

2. Vorrichtung gemäß Anspruch 1, wobei die Strahlungsquelle (12) zusätzlich mindestens einen Infrarotstrahler, mindestens eine Terahertz-Strahlungsquelle, und/oder mindestens eine LED-Einheit umfasst.

3. Vorrichtung gemäß Anspruch 1 oder 2, wobei die Detektoreinheit (15) mindestens ein Gaschromatographie-Ionenmobilitätsspektrometer umfasst.

4. Vorrichtung gemäß einem der Ansprüche 1 bis 3, die zudem eine regelbare Gaszuleitung (14a) umfasst, die dazu eingerichtet ist, das Messvolumen (30) mit einem Versuchsgas zu spülen.

5. Vorrichtung gemäß einem der vorhergehenden Ansprüche, die Teil eines mobilen, tragbaren Handgeräts ist.

6. Verfahren zur Oberflächenuntersuchung von Faserverbundkunststoff-Bauteilen, das umfasst:
Ausbilden eines Messvolumens (30), das an eine Probenfläche (100) eines Faserverbundkunststoff-Bauteils (20) angrenzt, durch eine Messglocke (10), die dazu eingerichtet ist, um das Faserverbundkunststoff-Bauteil (20) herum oder an dem Faserverbundkunststoff-Bauteil (20) anliegend das Messvolumen (30) auszubilden, das an die Probenfläche (100) angrenzt, wobei die Messglocke (10) eine Abdichtung (11) umfasst, die das Messvolumen (30) an einem Übergang zwischen der Messglocke (10) und der Probenfläche (100) abdichtet,
ein Bestrahlen der Probenfläche (100) des Faserverbundkunststoff-Bauteils (20) mit einer Strahlungsquelle (12), die dazu eingerichtet ist, mindestens einen schwerflüchtigen Stoff (22a) zumindest teilweise in Gas umzuwandeln; und
ein qualitatives und/oder quantitatives Erfassen des mindestens einen in Gas umgewandelten Stoffs (22b, 22c, 23) mittels einer Detektoreinheit (15), wobei durch die Strahlungsquelle (12) die Probenfläche (100) durch ein energiedurchlässiges Einkoppelfenster (13) in der Messglocke (10) hindurch bestrahlt wird, wobei die Messglocke (10) ein solches Einkoppelfenster (13) umfasst,
die Vorrichtung (1) eine Prozessüberwachungseinheit (17) umfasst, durch welche eine an der Probenfläche (100) herrschende Temperatur erfasst wird und wobei die Strahlungsquelle (12) einen Regler umfasst, durch den die erfasste Temperatur mit einem vorgegebenen Schwellwert verglichen wird, welcher eine Gefahr einer thermischen Schädigung der Probefläche (100) impliziert und durch das Bestrahlen bei Überschreiten der Temperatur über den Schwellwert beendet oder in Intensität reduziert wird, und wobei
die Strahlungsquelle (12) mindestens einen Laser und/oder mindestens einen Plasmastrahler umfasst,
**dadurch gekennzeichnet, dass**
die Strahlungsquelle (12) dazu eingerichtet ist, einen Abtrag (20a) von Material des Faserverbundkunststoff-Bauteils aus der Probenfläche (100) herauszulösen.

7. Verfahren gemäß Anspruch 6, wobei das Bestrahlen zusätzlich mit mindestens einem Infrarotstrahler, mindestens einer Terahertz-Strahlungsquelle, und/oder mindestens einer LED-Einheit erfolgt, der/die zusätzlich von der Strahlungsquelle (12) umfasst ist.

8. Verfahren gemäß Anspruch 6 oder 7, wobei das Erfassen mit mindestens einem Gaschromatographie-Ionenmobilitätsspektrometer erfolgt, das die Detektoreinheit (15) umfasst.

9. Verfahren gemäß einem der Ansprüche 6 bis 8, das ein Spülen des an die Probenfläche angrenzenden Messvolumens (30) mit einem Versuchsgas umfasst.

10. Verfahren gemäß einem der Ansprüche 6 bis 9, wobei das Bestrahlen im Rahmen einer Vorbehandlungsmethode zur Reinigung und/oder Aktivierung der Probenfläche (100) erfolgt, und wobei das Erfassen des mindestens einen in Gas umgewandelten Stoffs ein Erfassen von während der Vorbehandlungsmethode auftretendem Abgas (23) umfasst.

## Claims

1. Apparatus (1) for investigating the surface of fibre composite plastic components, comprising:
a radiation source (12), which is configured to convert at least one non-volatile substance (22a) at least partially into gas by irradiating a sample surface (100) of a fibre composite plastic component (20);
a detector unit (15), which is configured to qualitatively and/or quantitatively detect the at least one substance (22b, 22c, 23) converted into gas, wherein the apparatus also comprises a measuring bell (10), which is configured to form around the fibre composite plastic component or adjacent to the fibre composite plastic component a measuring volume (30), which adjoins the sample surface (100), wherein the measuring bell comprises a seal (11) of a flexible material, which seals the measuring volume at a transition between the measuring bell (10) and the sample surface (100),
wherein the radiation source (12) is configured to irradiate the sample surface (100) through an energy-permeable coupling-in window (13) in the measuring bell (10),
the measuring bell (10) comprises such a coupling-in window (13),
wherein the apparatus (1) comprises a process monitoring unit (17), which is configured to detect a temperature prevailing at the sample surface (100), and wherein the radiation source (12) comprises a controller, which is configured to compare the detected temperature with a predetermined threshold value, which implies a risk of thermal degradation of the sample surface (100), and to end or reduce the intensity of the irradiation if the temperature exceeds the threshold value, comprises, and wherein
the radiation source (12) comprises at least one laser and/or at least one plasma emitter,
**characterized in that**
the radiation source (12) is configured to initiate a removal (20a) of material of the fibre composite plastic component from the sample surface (100).

2. Apparatus according to Claim 1, wherein the radiation source (12) additionally comprises at least one infrared emitter, at least one terahertz radiation source, and/or at least one LED unit.

3. Apparatus according to Claim 1 or 2, wherein the detector unit (15) comprises at least one gas chromatography ion mobility spectrometer.

4. Apparatus according to one of Claims 1 to 3, which also comprises a controllable gas feed line (14a), which is configured to flush the measuring volume (30) with a test gas.

5. Apparatus according to one of the preceding claims, which is part of a mobile, portable hand-held device.

6. Method for investigating the surface of fibre composite plastic components, comprising:
forming a measuring volume (30), which adjoins the sample surface (100) of a fibre composite plastic component (20), by a measuring bell (10), which is configured to form around the fibre composite plastic component (20) or adjacent to the fibre composite plastic component (20) the measuring volume (30), which adjoins the sample surface (100), wherein the measuring bell (10) comprises a seal (11), which seals the measuring volume (30) at a transition between the measuring bell (10) and the sample surface (100),
irradiating the sample surface (100) of the fibre composite plastic component (20) with a radiation source (12), which is configured to convert at least one non-volatile substance (22a) at least partially into gas; and
qualitatively and/or quantitatively detecting the at least one substance (22b, 22c, 23) converted into gas by means of a detector unit (15), wherein the sample surface (100) is irradiated by the radiation source (12) through an energy-permeable coupling-in window (13) in the measuring bell (10), wherein the measuring bell (10) comprises such a coupling-in window (13),
the apparatus (1) comprises a process monitoring unit (17), by which a temperature prevailing at the sample surface (100) is detected and wherein the radiation source (12) comprises a controller, by which the detected temperature is compared with a predetermined threshold value, which implies a risk of thermal degradation of the sample surface (100), and by the irradiation is ended or reduced in intensity if the temperature exceeds the threshold value, and wherein
the radiation source (12) comprises at least one laser and/or at least one plasma emitter,
**characterized in that**
the radiation source (12) is configured to initiate a removal (20a) of material of the fibre composite plastic component from the sample surface (100).

7. Method according to Claim 6,
wherein the irradiation is additionally performed by at least one infrared emitter, at least one terahertz radiation source, and/or at least one LED unit, which is additionally comprised by the radiation source (12).

8. Method according to Claim 6 or 7,
wherein the detection is performed by at least one gas chromatography ion mobility spectrometer, which the detector unit (15) comprises.

9. Method according to one of Claims 6 to 8, which comprises flushing the measuring volume (30) adjoining the sample surface with a test gas.

10. Method according to one of Claims 6 to 9, wherein the irradiation is performed as part of a pretreatment method for cleaning and/or activating the sample surface (100), and wherein the detection of the at least one substance converted into gas comprises detecting exhaust gas (23) occurring during the pretreatment method.

## Revendications

1. Dispositif (1) d'examen de la surface d'éléments structuraux en matière plastique renforcée par des fibres, comprenant :
une source de rayonnement (12) qui est conçue pour, par irradiation d'une surface d'échantillon (100) d'un élément structural en matière plastique renforcée par des fibres (20), convertir au moins partiellement en gaz au moins une substance peu volatile (22a) ;
une unité de détection (15) qui est conçue pour détecter qualitativement et/ou quantitativement l'au moins une substance (22b, 22c, 23) convertie en gaz, le dispositif comportant en outre une cloche de mesure (10), laquelle est conçue pour former autour de l'élément structural en matière plastique renforcée par des fibres, ou en appui contre l'élément structural en matière plastique renforcée par des fibres, un volume de mesure (30) qui est voisin de la surface d'échantillon (100), la cloche de mesure comportant une garniture d'étanchéité (11) en un matériau flexible qui rend le volume de mesure étanche au niveau d'une transition entre la cloche de mesure (10) et la surface d'échantillon (100),
la source de rayonnement (12) étant conçue pour irradier la surface d'échantillon (100) à travers une fenêtre d'introduction (13) laissant passer l'énergie dans la cloche de mesure (10), la cloche de mesure (10) comportant une telle fenêtre d'introduction (13),
le dispositif (1) comportant une unité de surveillance de processus (17) qui est conçue pour acquérir une température qui règne au niveau de la surface d'échantillon (100) et la source de rayonnement (12) comportant un régulateur qui est conçu pour comparer la température acquise avec une valeur de seuil prédéfinie qui implique un danger d'endommagement thermique de la surface d'échantillon (100) et mettre fin à l'irradiation dans le cas où la température devient supérieure à la valeur de seuil ou réduire son intensité, comprend, et
la source de rayonnement (12) comportant au moins un laser et/ou au moins un projecteur de plasma,
**caractérisé en ce que**
la source de rayonnement (12) est conçue pour extraire un enlèvement (20a) de la matière de l'élément structural en matière plastique renforcée par des fibres depuis la surface d'échantillon (100).

2. Dispositif selon la revendication 1, la source de rayonnement (12) comportant en plus au moins un projecteur d'infrarouges, au moins une source de rayonnements dans la gamme du Térahertz et/ou au moins une unité à LED.

3. Dispositif selon la revendication 1 ou 2, l'unité de détection (15) comportant au moins un spectromètre à mobilité ionique à chromatographie en phase gazeuse.

4. Dispositif selon l'une des revendications 1 à 3, qui comporte en outre une conduite d'arrivée de gaz (14a), laquelle est conçue pour rincer le volume de mesure (30) avec un gaz d'essai.

5. Dispositif selon l'une des revendications précédentes, qui fait partie d'un appareil manuel portatif mobile.

6. Procédé d'examen de la surface d'éléments structuraux en matière plastique renforcée par des fibres, comprenant :
la formation d'un volume de mesure (30), qui est voisin d'une surface d'échantillon (100) d'un élément structural en matière plastique renforcée par des fibres (20), par une cloche de mesure (10) qui est conçue pour former autour de l'élément structural en matière plastique renforcée par des fibres (20), ou en appui (20) contre l'élément structural en matière plastique renforcée par des fibres (20), le volume de mesure (30) qui est voisin de la surface d'échantillon (100), la cloche de mesure (10) comportant une garniture d'étanchéité (11) qui rend le volume de mesure (30) étanche au niveau d'une transition entre la cloche de mesure (10) et la surface d'échantillon (100),
une irradiation de la surface d'échantillon (100) de l'élément structural en matière plastique renforcée par des fibres (20) avec une source de rayonnement (12) qui est conçue pour convertir au moins partiellement en gaz au moins une substance peu volatile (22a) ; et
une détection qualitative et/ou quantitative de l'au moins une substance (22b, 22c, 23) convertie en gaz au moyen d'une unité de détection (15), la surface d'échantillon (100) étant irradiée par la source de rayonnement (12) à travers une fenêtre d'introduction (13) laissant passer l'énergie dans la cloche de mesure (10), la cloche de mesure (10) comportant une telle fenêtre d'introduction (13),
le dispositif (1) comportant une unité de surveillance de processus (17) par le biais de laquelle est acquise une température qui règne au niveau de la surface d'échantillon (100) et la source de rayonnement (12) comportant un régulateur par le biais duquel la température acquise est comparée avec une valeur de seuil prédéfinie qui implique un danger d'endommagement thermique de la surface d'échantillon (100) et par l'irradiation est stoppée ou son intensité réduite dans le cas où la température devient supérieure à la valeur de seuil, et
la source de rayonnement (12) comportant au moins un laser et/ou au moins un projecteur de plasma,
**caractérisé en ce que**
la source de rayonnement (12) est conçue pour extraire un enlèvement (20a) de la matière de l'élément structural en matière plastique renforcée par des fibres depuis la surface d'échantillon (100).

7. Procédé selon la revendication 6, l'irradiation s'effectuant en plus avec au moins un projecteur d'infrarouges, au moins une source de rayonnements dans la gamme du Térahertz et/ou au moins une unité à LED qui est compris en plus de la source de rayonnement (12) .

8. Procédé selon la revendication 6 ou 7, la détection s'effectuant avec au moins un spectromètre à mobilité ionique à chromatographie en phase gazeuse qui comprend l'unité de détection (15).

9. Procédé selon l'une des revendications 6 à 8, qui comprend un rinçage du volume de mesure (30) voisin de la surface d'échantillon avec un gaz d'essai.

10. Procédé selon l'une des revendications 6 à 9, l'irradiation étant effectuée dans le cadre d'une méthode de prétraitement destinée à nettoyer et/ou à activer la surface d'échantillon (100), et la détection de l'au moins une substance convertie en gaz comprenant une détection des gaz rejetés (23) apparus pendant la méthode de prétraitement.
